# EUROPEAN PATENT APPLICATION

(11) **EP 4 707 298 A2**
(43) Date of publication of application: **11.03.2026**
(21) Application number: 24736303.9
(22) Date of filing: 03.05.2024
(51) Int. Cl.: C07K 16/22, A61P 35/00, A61P 43/00, G01N 33/60, G01N 33/68, A61K 39/00

(54) **HUMAN ANTI-FGF2 MONOCLONAL ANTIBODY (MAB), PHARMACEUTICAL COMPOSITION COMPRISING SAME, USE THEREOF AND KIT FOR THE DETECTION OF CANCER IN A BIOLOGICAL SAMPLE COMPRISING SAID MAB**

(30) Priority: 05.05.2023 BR 102023008634
(71) Applicant: INSTITUTO BUTANTAN, 05503900 São Paulo - SP (BR)
(72) Inventor: MORO, Ana Maria, 05503-900 São Paulo - SP (BR); MORAES, Jane Zveiter De, 04044-020 São Paulo - SP (BR); TSURUTA, Lilian Rumi, 05503-900 São Paulo - SP (BR); MAGALHAES, Carolina Georg, 96020-220 Pelotas - RS (BR); CHAMMAS, Roger, 01246-903 São Paulo (BR)
(74) Representative: Maiwald GmbH
(86) International application number: PCT/BR2024/050181
(87) International publication number: WO 2024/229542

(57) **Abstract**

The present invention refers to a human anti-FGF2 monoclonal antibody (mAb) comprising the amino acid sequence of the light chain as set in the SEQ ID NO: 1 or 3, and the amino acid sequence of the heavy chain as set in the SEQ ID NO: 2 or 4, Additionally, the present invention refers to a Pharmaceutical composition comprising said mAb and to its use in the production of a medicament to treat cancer, Additionally, the present invention refers to a kit for the detection of FGF2 protein in a biological sample comprising said mAb or a biologically active fragment thereof bound to a detectable fraction and can be used for prognosis or targeting cancer treatment, In addition to the therapeutic use associated with cancers, such as inhibition of angiogenesis, there are potential applications in the control of fibrotic diseases, neutralizing the excessive action of FGF, for example in diseases of intense tissue repair (keloids), and idiopathic fibrosis or secondary fibrosis to chronic inflammatory diseases, In addition to their therapeutic potential, antibodies can be used for immunoscintigraphy, labeled with radioisotopes, for molecular imaging and prediction of fibrosing responses.

## Description

### Field of application:

The present invention falls within the field of preparations for medical purposes, more specifically, in the area of medicinal preparations containing antigens or antibodies, as they refer to human anti-FGF2 monoclonal antibodies (mAbs).

### Background of the Invention and State of the Art:

Fibroblast growth factor 2 (FGF2) is considered a key protein in the development of melanoma and is responsible for playing a key role in tumor growth, angiogenesis, and metastasis.

Melanoma is the most aggressive and lethal type of cancer. In advanced stages, melanomas are difficult to treat because the tumors develop resistance to most therapies. Thus, a poor prognosis still persists for patients with metastatic melanoma, with a life expectancy of five years for 5 to 19 % of cases and an average survival of 5.3 months, despite the most recent therapeutic attempts.

In view of the technical problem, it is necessary to develop new treatment options, especially more effective ones, or ones that can be combined in the fight against this neoplasm. Inhibiting FGF2-mediated FGFR signaling could be an alternative treatment for patients with advanced or refractory melanoma, because FGF2 not only stimulates angiogenesis, but also directly increases the proliferation of melanoma cells.

In order to solve the existing technical problem, the present invention proposes human anti-FGF2 monoclonal antibodies (mAbs) capable of reducing FGF2-dependent cell proliferation and migration in human endothelial (HUVEC) and melanoma (SK-Mel-28) cells.

Fully human antibodies are currently considered the best option for therapy as they present the lowest risk of immunogenicity. Therefore, the strategy of the present invention to obtain human anti-FGF2 antibodies was the cloning of the VL and VH region of the functional human LC and Fd chains selected during the humanization process by guided selection (*phage display*) into vectors containing the genes of the constant region of the human immunoglobulin chains γ1HC, κLC or λLC for the expression of antibodies in IgG1 format.

This combination of strategies enabled the selection of the human anti-FGF2 mAbs of the present invention, which bind to the FGF2 protein and therefore have the potential to treat diverse types of cancer with expected success, including melanoma.

Some state of the art documents describe the development of human anti-FGF2 mAbs.

Brazilian patent application no. PI 0912035-1 A2, published on May 23, 2009, in the name of GALAXY BIOTECH, LLC., with the title: "MONOCLONAL ANTIBODY, PHARMACEUTICAL COMPOSITION, USE OF A PHARMACEUTICAL COMPOSITION, MOUSE MAB IN CHIMERIC OR HUMANIZED FORM, AND HUMANIZED ANTIBODY" refers to the combination of a monoclonal antibody (mAb) and recombinant DNA technologies to develop new biologicals, more specifically, the production of mAbs that bind and neutralize FGF2, that is, anti-FGF2 mAbs, in which said mAbs are humanized and obtained through the guided selection technique. However, the document distances itself from the present invention, since the present invention proposes mAbs that have only 55 % and 46 % identity with the light chain sequences of said document, and only 35 % and 65 % identity with the heavy chain sequences of said document.

The international patent application no. PCT/US1999/11844, published under the no. WO 00/03245 (A1) on January 20, 2000, in the name of CHUGAI PHARMACEUTICAL COMPANY LIMITED, with the title "PEPTIDE LIGANDS FOR THE HUMAN FIBROBLAST GROWTH FACTOR (FGF) RECEPTOR" relates to clones isolated from phage display libraries that bind to a human fibroblast growth factor (FGF) receptor probe. Polypeptides unrelated to the primary structure of FGF and encoded by DNA sequences of the clones obtained by the guided selection technique that bind to the FGF receptor are disclosed. Differently, the present invention discloses human anti-FGF2 mAbs comprising novel heavy chain and light chain sequences and providing new, more effective treatment options.

The US patent application no. US 2020/0291120 (A1), published on September 17, 2020, in the name of ZUMUTOR BIOLOGIES INC., with the title: "ANTI-CLEC2D ANTIBODIES AND METHODS OF USE THEREOF" describes anti-CLEC2D antibodies and related compositions and methods of using them, in which these antibodies are used as therapeutics and in diagnostic and prognostic applications in various types of cancer and other diseases. Differently, the present invention proposes human anti-FGF2 mAbs.

The article titled "Blocking FGF2 with a new specific monoclonal antibody impairs angiogenesis and experimental metastatic melanoma, suggesting a potential role in adjuvant settings", published in Cancer Letters; 371(2): 151 - 60, on February 28, 2016, under doi: 10.1016/j.canlet.2015.11.030, in the name of Aguiar RB et al. describes the therapeutic use of an anti-FGF2 monoclonal antibody (mAb), 3F12E7, using the B16-F10 melanoma model *in vivo.* In contrast, the present invention proposes human anti-FGF2 mAbs.

Thus, it is important to emphasize that the clinical efficiency of therapeutic antibodies depends on two types of functional characteristics: specific binding to the antigen conferred mainly by the complementarity determining regions (CDRs) within the VH and VL regions and effector functions mediated by the Fc.

Each variable region (VH and VL) has 3 CDRs, which form an antigen-binding structure. The difference in specificity of each antibody is mainly due to differences in these regions.

Thus, the amino acid sequences of the CDRs of the mabs clones of the present invention were compared to the amino acid sequence of the CDRs of the murine clone with regard to identity and similarity. Furthermore, according to epitope prediction by molecular docking, the human anti-FGF2 mAbs of the present invention interact with FGF2 in a residue region similar to the chimeric anti-FGF2 antibody, suggesting that they recognize the same binding site on the antigen (epitope).

The human anti-FGF2 mAbs of the present invention interact with FGF2 at residues Arg120 and Tyr124, and Lys119, Gln123, and Tyr124 respectively, within the heparin-binding domain (heparan sulfate proteoglycans - HSPG) of FGF2. This region is important in the functionality of FGF2, since in order to have its function, FGF2 interacts with FGFR and HSPG in a ternary complex (FGF2-FGFR-HSPG) resulting in the activation of signaling pathways involved mainly in the induction of cell proliferation, migration, and differentiation. Thus, the binding of human anti-FGF2 mAbs to the heparin-binding domain (HSPG) can block the activation of the signaling cascade mediated by the FGF2-FGFR-HSPG complex. Furthermore, the maintenance of the epitope in the humanization process indicates that the specificity of the human anti-FGF2 mAbs has been preserved, and that the antibodies of the present invention should bind to the FGF2 antigen in the same region as the murine anti- FGF2 parental antibody which has previously been characterized *in vitro* and *in vivo* showing promising results for use as an adjuvant in melanoma therapy.

Thus, although the development of anti-FGF2 mAbs through the guided selection technique already exists in the state of the art, none of the documents discloses the mAbs of the present invention and their therapeutic potential.

### Summary of the invention:

As already mentioned, the present invention will provide significant advantages for the development of new treatment options, especially more effective ones, or ones that can be combined in the fight against melanoma.

In a first aspect, the present invention refers to a human anti-FGF2 monoclonal antibody (mAb) comprising an antibody heavy chain and an antibody light chain, wherein said chains comprise complementarity determining regions (CDRs) selected from (i) - (ii):(i) CDR1, CDR2 and CDR3 regions of the light chain with the amino acid sequences of the positions 27 - 37, positions 55 - 57 and positions 94 - 102 of the SEQ ID NO: 1, respectively; and CDR1, CDR2 and CDR3 regions of the heavy chain with the amino acid sequences of the positions 26 - 35, positions 53 - 59 and positions 98 - 114 of the SEQ ID NO: 2, respectively; or (ii) CDR1, CDR2 and CDR3 regions of the light chain with the amino acid sequences of the positions 26 - 33, positions 51 - 53 and positions 90 - 101 of the SEQ ID NO: 3, respectively; and CDR1, CDR2 and CDR3 regions of the heavy chain with the amino acid sequences of the positions 26 - 33, positions 51 - 58 and positions 97 - 110 of the SEQ ID NO: 4, respectively.

In a second aspect, the present invention refers to a pharmaceutical composition comprising said mAb.

In a third aspect, the present invention refers to the use of said mAb for the production of a medicament to treat cancer.

In a fourth aspect, the present invention refers to a kit for the detection of the FGF2 protein in a biological sample comprising said mAb or a biologically active fragment thereof bound to a detectable fraction and can be used for prognosis or targeting cancer treatment.

In a fifth aspect, the present invention refers to the use of said mAb for the production of a medicament to control fibrotic diseases by neutralizing the excessive action of FGF, for example in diseases of intense tissue repair (keloids), and idiopathic fibrosis or fibrosis secondary to chronic inflammatory diseases.

In a sixth aspect, the present invention refers to the use of these mAbs to be applied for immunoscintigraphy, labeled with radioisotopes, for molecular imaging and prediction of fibrosing responses.

### Brief description of the figures:

The present invention, along with its additional advantages, can be better understood by referring to the attached figures and the following description.
Figures 1 and 2 present the experimental flow for humanization of the anti-FGF2 monoclonal antibody by guided selection (*phage display*).
Figure 3 graphically shows the binding assay of human anti-FGF2 antibodies to the rFGF2 antigen by SPR.
Figures 4A-B graphically show the assessment of HUVEC (A) and SK-Mel-28 (B) cell proliferation in the presence of human anti-FGF2 antibodies.
Figures 5A-D graphically show the assessment of the action of human anti-FGF2 antibodies on the migration of HUVEC (A) and SK-Mel-28 (C) cells, where (B) refers to the representative images of the HUVEC cell migration assay, and (D) refers to the representative images of the SK-Mel-28 cell migration assay.

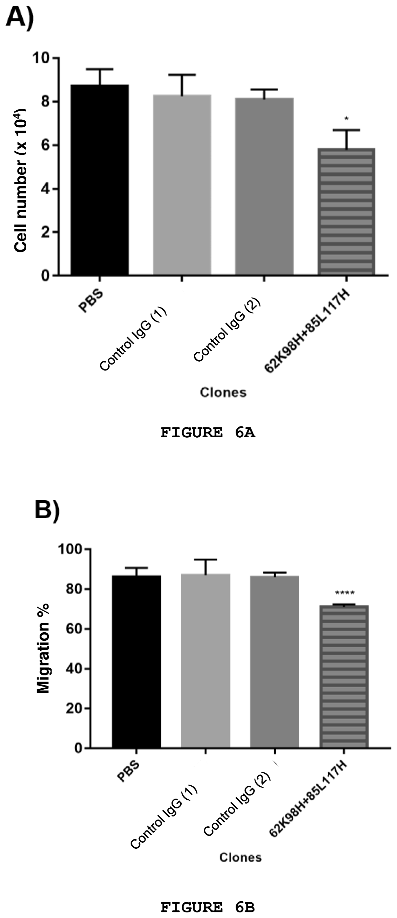

Figures 6A-C graphically show the assessment of the joint action of the human anti-FGF2 antibodies 62K98H and 85L117H on the proliferation and migration of SK-Mel-28 cells, where (A) refers to the number of viable SK-Mel-28 cells 72 h after treatment with 20 µg / mL of the human anti- FGF2 antibodies 62K98H and 85L117H, irrelevant human antibodies, or PBS; (B) refers to percentage migration of SK-Mel-28 cells after 48 h of treatment with 20 µg / mL of the human anti-FGF2 antibodies 62K98H and 85L117H, irrelevant human antibodies, or PBS; and (C) are representative images of the SK-Mel-28 cell migration assay.
Figures 7A-C illustrate the prediction of the binding of human (and chimeric) anti-FGF2 mAbs to the FGF2 protein, where (A) refers to the chimeric antibody, (B) refers to the human antibody 62K98H; and (C) refers to the human antibody 85L117H.

### Detailed description of the invention:

Although the present invention may be susceptible to different embodiments, a preferred embodiment is shown in the following detailed discussion, with the understanding that the present embodiment is to be considered an exemplification of the principles of the invention and is not intended to limit the present invention to what has been described in this report.

The present invention refers to a human anti-FGF2 monoclonal antibody (mAb) comprising an antibody heavy chain and an antibody light chain, wherein said chains comprise complementarity determining regions (CDRs) selected from (i) to (ii):
(i) CDR1, CDR2 and CDR3 regions of the light chain with the amino acid sequences of the positions 27 - 37, positions 55 - 57 and positions 94 - 102 of the SEQ ID NO: 1, respectively; and CDR1, CDR2 and CDR3 regions of the heavy chain with the amino acid sequences of the positions 26 - 35, positions 53 - 59 and positions 98 - 114 of the SEQ ID NO: 2, respectively; or
(ii) CDR1, CDR2 and CDR3 regions of the light chain with the amino acid sequences of the positions 26 - 33, positions 51 - 53 and positions 90 - 101 of the SEQ ID NO: 3, respectively; and CDR1, CDR2 and CDR3 regions of the heavy chain with the amino acid sequences of the positions 26 - 33, positions 51 - 58 and positions 97 - 110 of the SEQ ID NO: 4, respectively.

In one embodiment, such human anti-FGF2 monoclonal antibody (mAb) comprises the amino acid sequence of the light chain as set in the SEQ ID NO: 1 or 3, and the amino acid sequence of the heavy chain as set in the SEQ ID NO: 2 or 4.

**Table 1 - CDR1, CDR2 and CDR3 regions of the light chain of the monoclonal antibody of the present invention**

| SEQ ID NO | Name | Light chain aa sequence | CDR1 | CDR2 | CDR3 |
|---|---|---|---|---|---|
| 1 | VL_62K | DVVMTQSPLSLPVTLGQPASISCRS SQSLIYSDGNTRLYWFHQRPGQSPR RLIYGVSNRDSGVPDRFSGSGSGTD FTLKISRVEAEDVGVYYCMQSTHWP HTFGQGTKLEIK | **QSLIY SDGNTR** | **GVS** | **MQSTH WPHT** |
| 3 | VL_85 | QSVLTQPPSASGTPGQTVNISCSG ATSNIGGGSVNWYQQLPGAAPRLL IHSDNERASGVPDRFSGSKSGTSA SLAISGLQSEDEADYFCASWDDSL DGHVVFGGGTKVTVL | **TSNIG GGS** | **SDN** | **ASWDD SLDGH VV** |

**Table 2 - CDR1, CDR2 and CDR3 regions of the heavy chain of the monoclonal antibody of the present invention**

| SEQ ID NO | Name | Heavy chain aa sequence | CDR1 | CDR2 | CDR3 |
|---|---|---|---|---|---|
| 2 | VH_98H | QITLKESGPTLVKPTQTLTLTCT FSGFSLSTSGVGVGWIRQPPGEA LEWLAVIHWNEANYYSPYLRSRL TITKDTSKNQMVLTVTNMDPVDT ATYYCARGANSDSSGRLDAFDIW GQGTMVTVSS | **GFSLSTS GVG** | **IHWNEAN** | **ARGANSD SSGRLDA FDI** |
| 4 | VH_117 | QVQLVQSGAELKRPGSSVKVSCR ASGDTFKNYAITWVRQAPGQGLE WVGGIDPLVGVPNYTHRFQGRVT ITADEPTSTTYMEFRDLTSDDTA VYYCARGVRDQHWSYFDSWGQGT LVTVSS | **GDTFKNY A** | **IDPLVGV P** | **ARGVRDQ HWSYFDS** |

Although not claimed, the process of obtaining said mAbs is fundamental to obtaining the essential characteristics of the monoclonal antibodies of the present invention.

Thus, the process of obtaining the aforementioned mAbs comprises the steps of:
a) Obtaining the cDNA from the anti-FGF2 hybridoma 3F12E7;
b) Amplifying the murine LC and Fd genes of the anti- FGF2 hybridoma 3F12E7;
c) Sequential cloning of the murine LC and Fd genes into a vector for the construction of the murine Fab combinatorial library;
d) Enriching the library obtained in step "c" against the rFGF2 antigen (*phage display, panning*);
e) Selecting the murine anti-FGF2 Fab clone (*phage* ELISA and ELISA);
f) Cloning the murine Fd gene from the selected murine Fab clone into a vector containing the human LC gene repertoire library for construction of the hybrid Fab combinatorial library;
g) Enriching human Fab combinatorial libraries against the rFGF2 antigen (*phage display, panning*);
h) Selecting the human anti-FGF2 Fab clones (*phage* ELISA and ELISA);
i) Cloning the human light chain region of the selected hybrid clones into a vector;
j) Cloning the human heavy chain region of the selected hybrid clones into a vector; and
k) Combining light chain and heavy chain vectors to generate human anti-FGF2 antibodies.

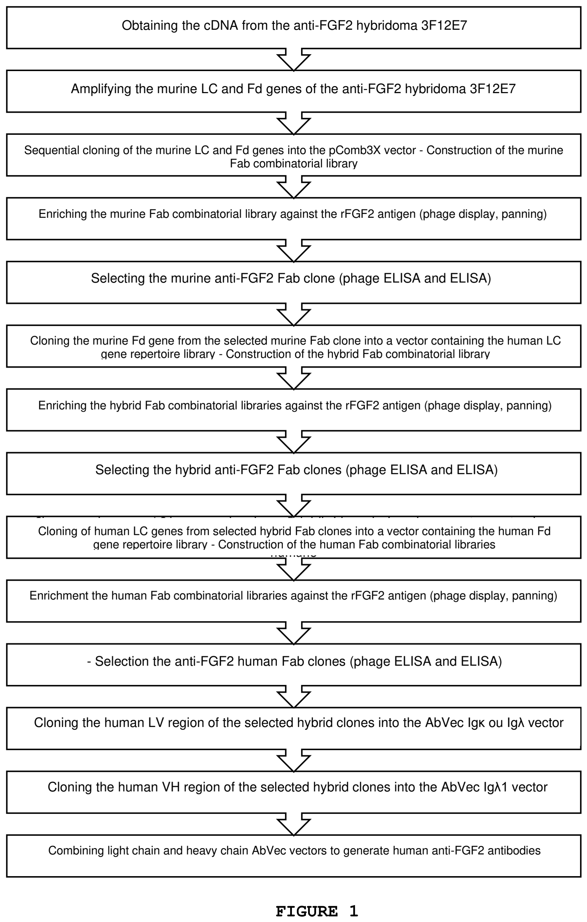

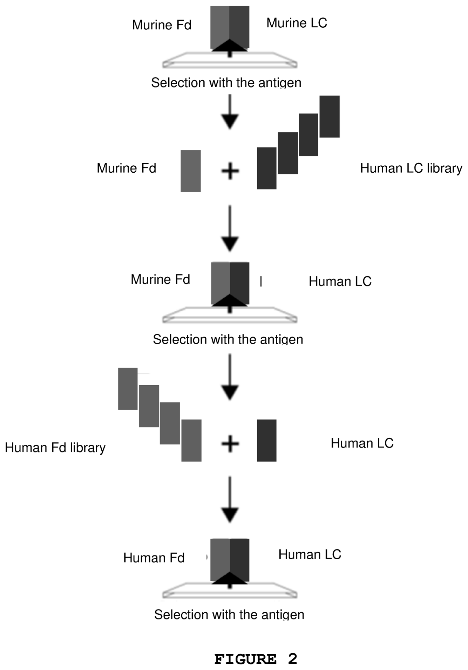

Figures 1 and 2 present the experimental flow for humanization of the monoclonal antibody anti-FGF2 by *phage display*.

In one embodiment, the anti-FGF2 hybridoma of 3F12E7 comprises the amino acid sequence of the light chain as set in the SEQ ID NO: 5, and the amino acid sequence of the heavy chain as set in the SEQ ID NO: 6.

**Table 3 - CDR1, CDR2 and CDR3 regions of the light chain of the anti-FGF2 hybridoma of 3F12E7**

| SEQ ID NO | Name | Light chain aa sequence | CDR1 | CDR2 | CDR3 |
|---|---|---|---|---|---|
| 5 | VL_3F12 E7 | DIELTQSPASLSASVGETVTGNIYS YLAWYQQKQGKSPQLLVYDAKTLAD GVPSRFTGSGSGTQFSLKINSLQPE DFGSYYCQHFWNTPRTFGGGTKLEI K | **GNIYS Y** | **DAK** | **QHFWNT PRT** |

**Table 4 - CDR1, CDR2 and CDR3 regions of the heavy chain of the anti-FGF2 hybridoma of 3F12E7**

| SEQ ID NO | Name | Heavy chain aa sequence | CDR1 | CDR2 | CDR3 |
|---|---|---|---|---|---|
| 6 | VH_3F1 2E7 | GELQESGPELVKPGASVKISCKA SGYSFTGYYMNWVKHSPEKSLEW IGEINPSTGGTSYNQKFKAKATL TVDKSSTTAYMQLKGLTSEDSAV YYCASGGYYEGFDYWGQGTTVTV SS | **GYSFTGY Y** | **INPSTGG T** | **ASGGYYE GFDY** |

In one embodiment, the vectors used in the process described above are AbVec vectors as described in the article entitled "Efficient generation of monoclonal antibodies from single human B cells by single cell RT-PCR and expression vector cloning", in the name of Tiller T et al, published on J Immunol Methods on January 1st, 2008; 329(1-2): 112-24, doi: 10.1016/j.jim.2007.09.017, available in https://www.addgene.org/browse/article/28189951/.

In one embodiment, for the establishment of permanent stable strains, the sequences of the variable regions of the light and heavy chains can be cloned into expression vectors for CHO cells, e.g., pCHO 3.0, or another used for transfection of Expi-CHO cells (Thermofisher) for stable expression of the antibody.

The mixed stable population cloned to obtain the monoclonal strain is carried out by limiting dilution or robotic method. The selection of the best clone is based on cell growth, productivity, stability, physicochemical characterization, and the results of *in vitro* tests (inhibition of cell proliferation and cell migration).

The selected clone is grown in culture medium appropriate for the host cell, in increasing volumes, to establish fed-batch cultivation. The cultivation mode can be fed batch, with an optional decrease in cultivation temperature.

After cultivation, the supernatant is clarified to remove cells and debris, and then purified in chromatographic steps, such as capture on protein A and chromatography on ion exchange resins. There are two viral removal / inactivation steps, by pH and membrane filtration.

The clarified supernatant can be purified using the conventional monoclonal antibody purification method, following protein A resin affinity chromatography, retention at acidic pH (viral inactivation), cation resin chromatography and anion resin chromatography. These steps allow the purification of monoclonal antibodies to almost 100 %, with the removal of residual DNA and residual proteins from the host cell.

Finally, the mAbs obtained are assessed for purity, structure, electrical charge, and power.

Additionally, the present invention refers to a pharmaceutical composition comprising a mAb comprising the amino acid sequence of the light chain as set in the SEQ ID NO: 1 or 3, and the amino acid sequence of the heavy chain as set in the SEQ ID NO: 2 or 4.

On one embodiment, the pharmaceutical composition comprises the mAb in a physiologically acceptable carrier, optionally with excipients or stabilizers, in the form of aqueous or lyophilized solutions.

Acceptable carriers, excipients or stabilizers are not toxic to the recipients at the dosages and concentrations used and optionally include buffers such as phosphate, citrate, or acetate at a pH typically of 5.0 to 8.0, most frequently 6.0 to 7.0; salts such as sodium chloride, potassium chloride, among others to make isotonic; antioxidants, preservatives, low molecular weight polypeptides, proteins, hydrophilic polymers such as polysorbate 80, amino acids, carbohydrates, chelating agents, sugars and other standard ingredients known to a person skilled in the art (Remington's Pharmaceutical Science, 16th edition, Osol, A. Ed. 1980). MAb is typically present at a concentration of 10 to 100 mg / ml, for example, 50 mg / ml.

Additionally, the present invention refers to the use of said mAb for the production of a proposed medicament for cancer treatment.

Melanoma was the type of cancer investigated in the preclinical model with the murine antibody. Furthermore, given that the increased expression of FGF2 is related to diverse types of cancer, the scope of the therapeutic potential of the anti-FGF2 mAbs object of the present invention could be extended.

Thus, in one embodiment of the invention, the cancer is selected from the group consisting of melanoma, pancreatic cancer, non-small cell lung carcinoma, prostate tumors, hepatocellular carcinoma, Lewis lung carcinoma, among other cancers related to increased FGF2 expression. Preferably, the cancer is melanoma.

Additionally, in addition to the therapeutic use associated with cancers, such as inhibition of angiogenesis, the present invention refers to the use of said mAb for the production of a medicament to control fibrotic diseases by neutralizing the excessive action of FGF, for example in diseases of intense tissue repair (keloids), and idiopathic fibrosis or fibrosis secondary to chronic inflammatory diseases.

Beyond the therapeutic expectation, the present invention refers to the use of said mAb for application in radioisotope-labeled immunoscintigraphy for molecular imaging and prediction of fibrosing responses.

Additionally, the present invention refers to a kit for the detection of the FGF2 protein in a biological sample comprising said mAb or a biologically active fragment thereof bound to a detectable fraction and that can be used for prognosis or targeting cancer treatment.

Therefore, in order to elucidate the present invention, in the following are presented experimental results and embodiments of the invention in order to highlight the inventive step of using the mAb of the present invention.

### Embodiments of the invention

The construction of human anti-FGF2 antibodies was carried out using the vectors AbVec (https://www.addgene.org/browse/article/28189951/) containing the genes of the constant region of the human immunoglobulin chains γlHC, κLC or λLC for antibody expression in HEK 293 or Expi-CHO mammalian cells (Thermofisher).

This system made it possible to combine each human LC with the different human HCs to produce human anti-FGF2 antibodies. During the humanization process by guided selection, three human LC / HC murine hybrids and eight human HC / LC murine hybrids were obtained. All the LC and HC variable regions were combined with each other for the transfection of the HC - LC pair, resulting in 24 possibilities.

The human VH and VL regions were amplified using specific primers according to the classification of the V and J families, and the amplified VL and VH regions were cloned into the respective vectors containing the constant region of the human immunoglobulin chains. The vectors obtained were used to transfect the LC and HC combinations into HEK 293 cells (Thermofisher, initially, and then ExpiCHO (Thermofisher) for the production of human anti-FGF2 antibodies. The CHO cell is the most widely used in the monoclonal antibody industry.

The antibodies were purified by affinity chromatography using a column with A-Sepharose protein resin, and the integrity of the antibodies was analyzed by electrophoresis (SDS-PAGE) and WB. Of the 24 HC - LC combinations, not all were productive.

The two mAbs with remarkable results were evaluated in relation to the epitope recognized on the FGF2 molecule, and a recognition site similar to the site recognized by the original murine antibody was found, expressed in chimeric form to contain the same Fc region as the human antibodies.

In one preferred embodiment of the invention, the two human anti-FGF2 antibodies 62K98H and 85L117H obtained can be expressed in expression vectors for CHO cells, such as the pCHO 3.0 vector for synthesis in the Expi- CHO cell. The cultivation mode can be fed batch, with an optional decrease in cultivation temperature. The clarified supernatant can be purified using the conventional method for purifying monoclonal antibodies, following the sequence of affinity chromatography on protein A resin, retention at acidic pH (viral inactivation), chromatography on cationic resin and chromatography on anionic resin. These steps allow the purification of monoclonal antibodies to almost 100 %, with the removal of residual DNA and residual proteins from the host cell.

### Experimental results

### - Binding assay of human anti-FGF2 antibodies to the rFGF2 antigen by SPR.

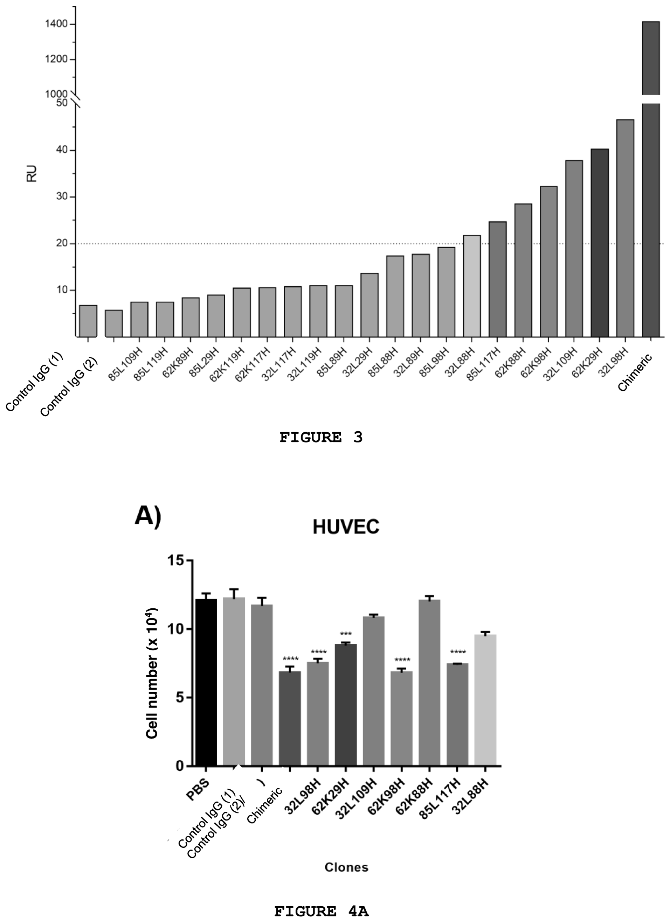

Antibodies at a concentration of 25 µg / mL were applied to the sensor immobilized with 1800 RU of rFGF2. The results show the RU values at the point of binding stability (Figure 3).

The kₐ, k_{d} and K_{D} values of the anti-FGF2 antibodies were determined by the kinetic affinity assay, as shown in Table 5 below:

**Table 5 - Kₐ, k_{d} and K_{D} values of anti-FGF2 antibodies determined by kinetic affinity assay.**

| **Antibody** | **kₐ (M⁻¹. s⁻¹)** | **k_{d} (s⁻¹)** | **K_{D} (M)** |
|---|---|---|---|
| Chimeric | 3,52x10⁵ | 6,11x10⁻⁵ | 1,74x10⁻¹⁰ |
| Murine 3F12E7 | 1,93x10⁵ | 7,96x10⁻⁵ | 4,13x10⁻¹⁰ |
| 32L98H | 3,82x10⁵ | 3,26x10⁻³ | 8,53x10⁻⁹ |
| 62K98H | 1,71x10⁵ | 1,56x10⁻³ | 9,10x10⁻⁹ |
| 32L109H | 1,85x10⁵ | 1,76x10⁻³ | 9,52x10⁻⁹ |
| 62K88H | 1,55x10⁵ | 1,66x10⁻³ | 1,07x10⁻⁸ |
| 85L117H | 4,92x10⁴ | 8,34x10⁻⁴ | 1,70x10⁻⁸ |
| 32L88H | 1,27x10⁵ | 2,50x10⁻³ | 1,97x10⁻⁸ |
| 62K29H | 4,11x10⁵ | 2,09x10⁻² | 5,09x10⁻⁸ |

Thus, as can be seen in Figure 3, the antibodies purified from 20 HC - LC combinations expressed as IgG were analyzed for their ability to bind to the antigen, which showed a variety of results demonstrating that not all pairs work in the same way.

As shown in Table 3, seven pairs of human HC- LC sequences with the highest antigen-binding capacity were selected for the functional experiments to inhibit the proliferation and migration of human, endothelial (HUVEC) and melanoma (SK-Mel-28) cells.

### - Evaluation of HUVEC and SK-Mel-28 cell proliferation in the presence of human anti-FGF2 antibodies:

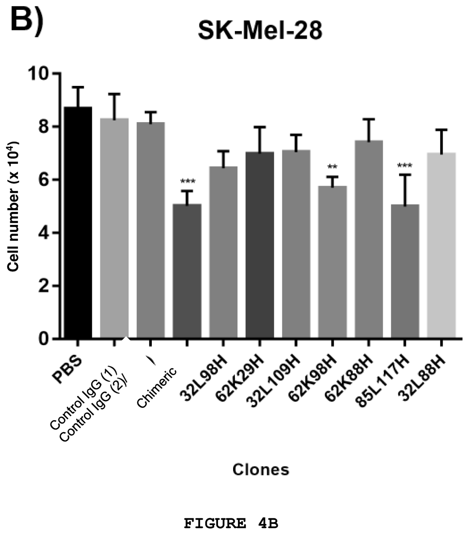

The number of viable HUVEC (Figure 4A) and SK-Mel-28 (Figure 4B) cells after 72 h of treatment with 20 µg / mL of the human and chimeric anti-FGF2 antibodies, irrelevant human antibodies, or PBS. The assay was performed by trypan blue dye exclusion.

ANOVA followed by the Bonferroni test was performed to verify the presence or absence of a significant difference between the groups treated with the anti-FGF2 antibodies and the control groups treated with irrelevant human antibodies (IgG control 1 and 2). ** p ≤ 0.01; *** p ≤ 0.001; **** p ≤ 0.0001.

The human anti-FGF2 antibodies 62K98H and 85L117H, as well as the chimeric anti-FGF2 antibody, were able to significantly reduce the number of viable cells compared to the groups treated with irrelevant human antibodies (IgG control 1 and 2) in the proliferation assays with the two cell lines, HUVEC and SK-Mel-28.

### - Evaluation of the action of human anti-FGF2 antibodies on the migration of HUVEC and SK-Mel-28 cells:

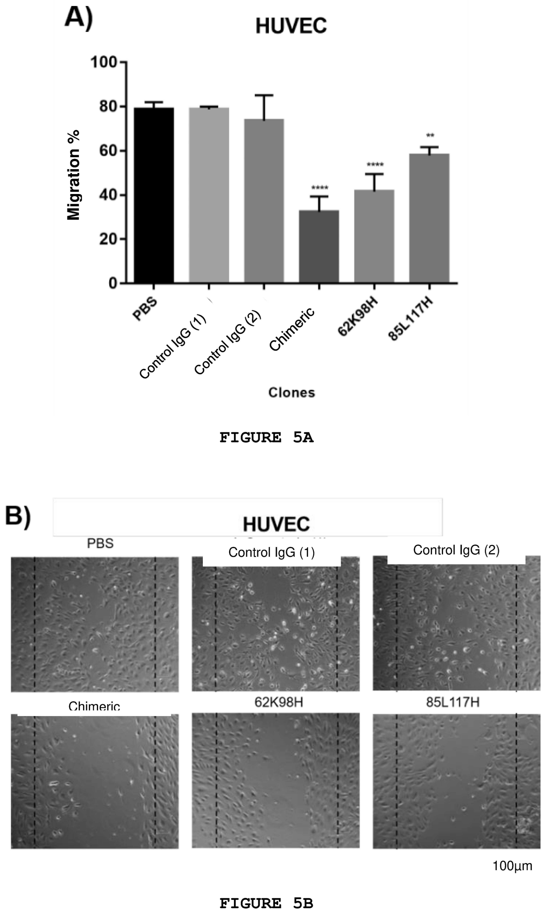

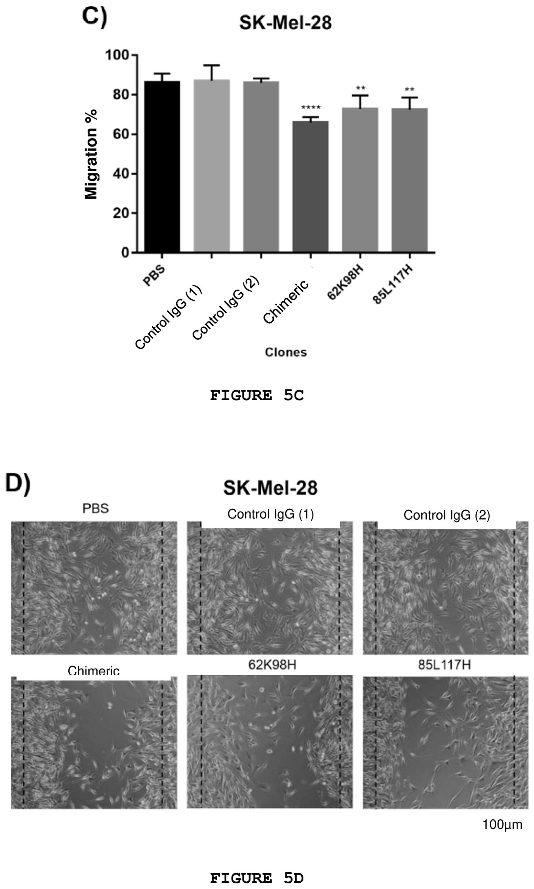

Figure 5 refers to the percentage of migration of HUVEC (Figure 5A) and SK-Mel-28 (Figure 5A-C) cells after 48 h of treatment with 20 µg / mL of human and chimeric anti- FGF2 antibodies, irrelevant human antibodies, or PBS.

ANOVA followed by the Bonferroni test was performed to verify the presence or absence of a significant difference between the groups treated with the anti-FGF2 antibodies and the control groups treated with irrelevant human antibodies (IgG control 1 and 2). p ≤ 0.01; **** p ≤ 0.0001.

Representative images of the HUVEC (Figure 5B) and SK-Mel-28 (Figure 5D) cell migration assay are also shown. The dashed lines indicate the cell-free area at time 0 h.

Here we demonstrate the ability of the human anti-FGF2 antibodies 62K98H and 85L117H to modulate cell migration in a cell-free area created in a confluent culture. It can be seen that in both cell lines the two human anti- FGF2 antibodies assessed, and the chimeric anti-FGF2 antibody, were able to significantly attenuate cell migration compared to the groups treated with irrelevant human antibodies (control IgG 1 and 2).

Molecules capable of inhibiting cell proliferation and migration events are a focus of interest for the development of cancer therapies, since, in endothelial cells, these processes are necessary for tumor dissemination, invasion and angiogenesis, and, in tumor cells, they are key steps in triggering metastasis and cancer progression.

### - Evaluation of the joint action of the human anti-FGF2 antibodies 62K98H and 85L117H on the proliferation and migration of SK-Mel-28 cells:

Figure 6A refers to the number of viable SK-Mel-28 cells 72 h after treatment with 20 µg / mL of the human anti-FGF2 antibodies 62K98H and 85L117H, irrelevant human antibodies, or PBS. The assay is performed by trypan blue dye exclusion.

Figure 6B refers to the percentage migration of SK-Mel-28 cells after 48 h of treatment with 20 µg / mL of the human anti-FGF2 antibodies 62K98H and 85L117H, irrelevant human antibodies, or PBS.

ANOVA followed by the Bonferroni test was performed to verify the presence or absence of a significant difference between the groups treated with the anti-FGF2 antibodies and the control groups treated with irrelevant human antibodies (control IgG 1 and 2). * p ≤ 0,05; **** p ≤ 0,0001.

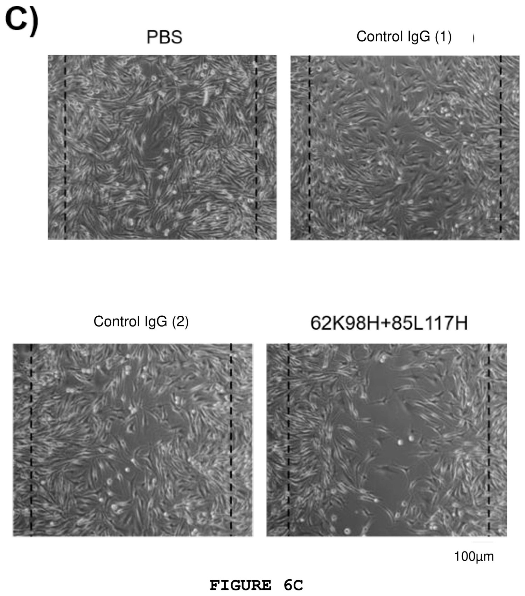

Figure 6C shows representative images of the SK-Mel-28 cell migration assay. The dashed lines indicate the cell-free area at time 0 h.

This test was carried out in order to verify the presence or absence of synergistic potential between the human anti-FGF2 antibodies 62K98H and 85L117H. The results show that no synergistic effect was observed between the two antibodies, with results similar to those found when the antibodies were assessed individually in the SK-Mel-28 cell proliferation and migration assay, as shown above.

### - Prediction of the binding of human (and chimeric) anti-FGF2 mAbs to the FGF2 protein:

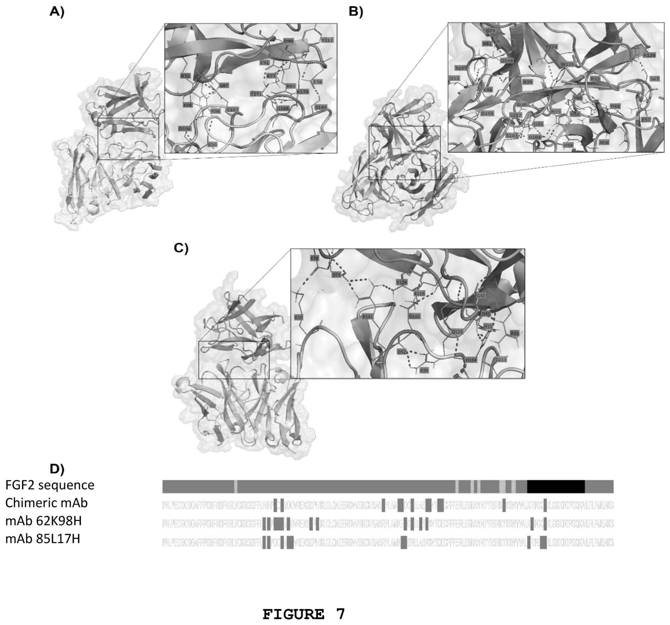

Figure 7 is a three-dimensional representation of the molecular complexes between the Fv region and FGF2, of the chimeric antibody (Figure 7A), of the human antibodies 62K98H (Figure 7B) and 85L117H (Figure 7C).

Residues that are part of the antigen- antibody interaction in FGF2 and the antibodies (found in LigPlot+ / DIMPLOT analyses) are represented by magenta and orange, respectively. Dashed lines indicate hydrogen bridges. Figure 7D shows a schematic representation in yellow of the FGF2 residues predicted to interact with the human and chimeric mAbs. The FGFR receptor and the heparin binding site of FGF2 are represented in gray and black, respectively.

Therefore, the results herein presented showed that, according to molecular prediction, the chimeric anti-FGF2 mAb would contact FGF2 at residues Tyr111 and Tyr124 within the FGFR and heparin receptor binding domains, while the human anti-FGF2 mAbs 62K98H and 85L117H would contact FGF2 at residues Arg120, Tyr124, and Lys119, Gln123, Tyr124, respectively, within the heparin binding domains. The inhibition of these domains in the FGF2 molecule by the human and chimeric anti-FGF2 mAbs may interfere with the function of FGF2 by impairing cell signaling and consequently reducing cell migration and proliferation, as was observed in the results shown above. In addition, it was predicted that the human anti-FGF2 mAbs 62K98H and 85L117H iterate with FGF2 in a region of residues similar to the chimeric anti-FGF2 mAb. This result suggests that the human anti-FGF2 mAbs recognize the same epitope as the chimeric anti-FGF2, indicating success in the humanization and generation of human antibodies corresponding to the murine anti-FGF2 mAb 3F12E7.

In short, the invention relates to the following aspects, as defined in the following numbered items:
1. Human anti-FGF2 monoclonal antibody (mAb) comprising an antibody heavy chain and an antibody light chain, wherein said chains comprise complementarity determining regions (CDRs) selected from (i) to (ii):
   (i) CDR1, CDR2 and CDR3 regions of the light chain with the amino acid sequences of the positions 27 - 37, positions 55 - 57 and positions 94 - 102 of the SEQ ID NO: 1, respectively; and CDR1, CDR2 and CDR3 regions of the heavy chain with the amino acid sequences of the positions 26 - 35, positions 53 - 59 and positions 98 - 114 of the SEQ ID NO: 2, respectively; and
   (ii) CDR1, CDR2 and CDR3 regions of the light chain with the amino acid sequences of the positions 26 - 33, positions 51 - 53 and positions 90 - 101 of the SEQ ID NO: 3, respectively; and CDR1, CDR2 and CDR3 regions of the heavy chain with the amino acid sequences of the positions 26 - 33, positions 51 - 58 and positions 97 - 110 of the SEQ ID NO: 4, respectively.
2. Human anti-FGF2 monoclonal antibody (mAb), according to item 1, comprising the amino acid sequence of the light chain as set in the SEQ ID NO: 1 or 3, and the amino acid sequence of the heavy chain as set in the SEQ ID NO: 2 or 4.
3. Pharmaceutical composition comprising a mAb as defined in item 1 or 2.
4. Pharmaceutical composition, according to item 3, comprising a physiologically acceptable carrier, optionally with excipients or stabilizers, in the form of aqueous or lyophilized solutions.
5. Pharmaceutical composition, according to items 2 or 3, wherein the mAb is typically present at a concentration of 10 to 100 mg / ml, preferably 50 mg / ml.
6. Use of mAb as defined in item 1 or 2 characterized by the fact that it is for the production of a drug to treat cancer.
7. Use of mAb as defined in item 1 or 2, in which it is for the production of a medicament to control fibrotic diseases by neutralizing the excessive action of FGF, preferably in diseases of intense tissue repair (keloids), and idiopathic fibrosis or fibrosis secondary to chronic inflammatory diseases.
8. Use of mAb as defined in item 1 or 2, where it is for application in immunoscintigraphy, labeled with radioisotopes, for molecular imaging and prediction of fibrosing responses.
9. Kit for detecting cancer in a biological sample comprising the mAb as defined in item 1 or 2, or a biologically active fragment thereof bound to a detectable fraction.
10. Kit, according to item 9, in which it is applied to prognosis or treatment targeting for cancer.

Thus, the embodiments presented in the present invention do not limit the totality of possibilities, and it will be understood that various omissions, substitutions, and alterations can be made by a person skilled in the art, without departing from the scope of the present invention.

It is expressly provided that all combinations of elements that perform the same function in substantially the same way to achieve the same results are within the scope of the invention. Substitutions of elements from one described embodiment to another are also fully intended and contemplated.

Those skilled in the art will appreciate the knowledge presented herein and will be able to reproduce the invention in the embodiments presented and in other variants covered by the scope of the claims.

## Claims

1. Human anti-FGF2 monoclonal antibody (mAb) **characterized by** the fact that it comprises an antibody heavy chain and an antibody light chain, wherein said chains comprise complementarity determining regions (CDRs) selected from (i) to (ii):
(i) CDR1, CDR2 and CDR3 regions of the light chain with the amino acid sequences of the positions 27 - 37, positions 55 - 57 and positions 94 - 102 of the SEQ ID NO: 1, respectively; and CDR1, CDR2 and CDR3 regions of the heavy chain with the amino acid sequences of the positions 26 - 35, positions 53 - 59 and positions 98 - 114 of the SEQ ID NO: 2, respectively; or
(ii) CDR1, CDR2 and CDR3 regions of the light chain with the amino acid sequences of the positions 26 - 33, positions 51 - 53 and positions 90 - 101 of the SEQ ID NO: 3, respectively; and CDR1, CDR2 and CDR3 regions of the heavy chain with the amino acid sequences of the positions 26 - 33, positions 51 - 58 and positions 97 - 110 of the SEQ ID NO: 4, respectively.

2. Human anti-FGF2 monoclonal antibody (mAb), according to claim 1, **characterized by** the fact that it comprises the amino acid sequence of the light chain as set in the SEQ ID NO: 1 or 3, and the amino acid sequence of the heavy chain as set in the SEQ ID NO: 2 or 4.

3. Pharmaceutical composition **characterized by** the fact that it comprises a mAb as defined in claim 1 or 2.

4. Pharmaceutical composition, according to claim 3, **characterized by** the fact that it comprises a physiologically acceptable carrier, optionally with excipients or stabilizers, in the form of aqueous or lyophilized solutions.

5. Pharmaceutical composition, according to claim 3 or 4, **characterized by** the fact that mAb is typically present at a concentration of 10 to 100 mg / ml, preferably, 50 mg / ml.

6. Use of the monoclonal antibody (mAb) as defined in claim 1 or 2 **characterized by** the fact that it is for the production of a medicament to treat cancer.

7. Use of the monoclonal antibody (mAb) as defined in claim 1 or 2 **characterized by** the fact that it is for the production of a medicament to control fibrotic diseases, neutralizing the excessive action of FGF, preferably, in diseases of intense tissue repair (keloids), and idiopathic fibrosis or secondary to chronic inflammatory diseases.

8. Use of the monoclonal antibody (mAb) as defined in claim 1 or 2 **characterized by** the fact that it is for application in immunoscintigraphy, labeled with radioisotopes, for molecular imaging and prediction of fibrosing responses.

9. Kit for detecting cancer in a biological sample comprising the monoclonal antibody (mAb) as defined in claim 1 or 2, or a biologically active fragment thereof **characterized by** the fact that said mAb or biologically active fragment thereof is bound to a detectable fraction.

10. Kit, according to claim 9, **characterized by** the fact that it is to be applied in the prognosis or treatment targeting for cancer.
